# EUROPEAN PATENT APPLICATION

(11) **EP 0 561 745 A2**
(43) Date of publication of application: **22.09.1993**
(21) Application number: 93830049.8
(22) Date of filing: 11.02.1993
(51) Int. Cl.: A23L 1/03, A23L 1/302, A23L 1/305, A61K 31/045, A61K 31/375, A61K 31/205

(54) **Dietary energy-giving product for subjects under physical exhaustion conditions**

(30) Priority: 20.03.1992 IT RM920205
(71) Applicant: SIRC S.p.A. NATURAL & DIETETIC FOODS, I-20090 Caleppio Di Settala (MI) (IT)
(72) Inventor: Littera, Renato, I-10143 Torino (IT)
(74) Representative: Sarpi, Maurizio

(57) **Abstract**

The use of the combination of carnitine, octocosanol, and vitamin C as dietary energy-giving product capable of favourably acting both on the muscular strength and the fatigue resistance and the cardiovascular function of a subject under prolonged physical strain.

## Description

The present invention relates to a dietary product obtained by the combination of three nutritional principles which are commonly present in several foods even if in a little amount: carnitine, octocosanol and vitamin C. The presence and the association thereof in a dietary product is justified by the metabolic and functional activity of the three above-mentioned substances and by their experimentally proved synergism providing a favourable action both on the muscular strength and the endurance of a subject and his cardiovascular function. Therefore, the product finds its main application in nourishing humans subjected to prolonged physical exercises or going in for a sport.

### NUTRITIONAL AND METABOLIC NEEDS OF A SPORTSMAN

The different sports are characterized by particular energy requirements.

During short and strong exercises (anaerobic-alacticide phase) energy requirements are basically formed of ATP (adenosine-triphosphate) and phosphocreatine. In case of strong but longer exercises (anaerobic-alacticide phase), in which lactic acid causes the muscle to incur a debt of oxygen, energy requirements are formed of muscular and hepatic glycogen. In case of endurance exercises (aerobic phase) energy requirements are above all supported by the oxidation of the fatty acids and ketone bodies. How true it is that the first energy support for muscular contraction is ATP produced both in the mitochondria and in the cell cytoplasm, it is also true that the amount of ATP which can accumulate in the muscle is quite little and sufficient only for 1-2 second activity. Therefore, ATP should be continously restored.

The regeneration of ATP depends on the metabolic oxidation of several substrate such as glucose, glycogen, fatty acids, ketone bodies and aminoacids. The muscle is then capable of using both glucides and lipids for energy development: the respective amounts of utilization depends on the type and length of the exercise. Thus, whilst the oxygen consumed by the individual at rest serves to oxidize above all the fatty acids, both the amount of oxygen consumed by the muscles and the carbohydrate oxidation increase during the exercises. Besides the necessary energetic substrates it is important to assure to the organism performing physical exercises also substances controlling and facilitating the availability and the oxidation rate of such substrates according to the energy demand of the muscle. This is the function of the three nutrients which the dietary product of the invention is composed of and now individually taken into consideration.

### OCTOCOSANOL

Octocosanol is a primary aliphatic alchool with 28 carbon atoms (originating its name from the Greek language) which can be found in a little amount in the wheat germ and in the surface wax of several fruits and leaves of common vegetables. Its chemical formula is as follows:

CH₃(CH₂)₂₆CH₂OH

and its molecular weight is 410.

Avocade oil and wheat germ oil are the most concentrated source of octocosanol (about 100-250 ppm) even if it can be produced by chemical synthesis today. It can be added to vegetal oils to be enriched. The raw material used in the preparation of such product contains 12% octocosanol. During experiments carried out with test animals and humans, ocotocosanol was proved capable of improving the physical performance, especially in endurance tests, through an increase both of the oxygen consumed and the amount of the glycogen reserves.

Moreover it was proved capable of improving the lipaemic picture through the reduction of the triglyceride and cholesterol rates in the blood.

Experiments with men have been conducted especially by CURETON of the Illinois University, who made researches on the effects of wheat germ oil to the physical efficiency and the capability of resistance to the reaction times and the cardiovascular functions as well, and individuated in the octocosanol the substance responsible for the noticed effects.

In double-blind tests conducted with American youth belonging to diver teams and subjected both to several tests of physical and cardiovascular performance and diets supplemented with wheat germ oil and octocosanol, he found an improvement of the physical fitness notcorrelated to the presence of vitamins.

The subjects were divided into three groups who received, respectively:
1. group: wheat germ oil containing 0.385 mg octocosanol and 0.880 mg vitamin E;
2. group: cotton seed oil added with 0.385 mg octocosanol nd 0.880 mg vitamin E;
3. group: cotton seed oil plus 0.880 mg vitamin E (octocosanol = 0 mg).

After a test of three weeks the first two groups were found having better physical form over the third group, especially as far as force and endurance tests is concerned.

Further endurance tests showed an improvement over the placebo as a result of the administration of wheat germ oil and octocosanol with an average of 12.51 points over 6.00 points of the placebo.

Also the cardiovascular function was improved as a result of the administration of octocosanol as shown by the greater height of the wave T in the electrocardiogram of experimental subjects (the height gain for octocosanol is 3.746 over 0.623 for placebo). Octocosanol was also proved capable of stabilizing the basal metabolism as a result of a stress.

In a number of subjects who were given wheat germ oil the increase in basal metabolism resulting from stress was really lower than that of placebo.

Further improvements have been noticed in experiments regarding the reaction time of the organism and the grasp firmness which have been increased after administration of octocosanol oil.

An improvement of the oxygen utilization and the fatigue resistance has also been noticed in some experiments with animals.

Actually 3 groups of rats put in a decompression chamber had a longer survival and a greater resistance to anoxia when their diet was supplemented with wheat germ oil and octocosanol than when using placebo (refined cotton oil). Most recent experiments with mice, the diet of which was supplemented with 0.3% octocosanol extracted from cane sugar, showed that mice had a more prolonged resistance to physical tests (swimming) than those which were not given octocosanol.

Moreover the group supplemented with octocosanol had a better lipidemic aspect than the group with a lower trigliceride and cholesterolemia level.

Instead, the amount of proteins in the liver of animals fed with octocosanol increased.

On the basis of experimental data the authors came to the conclusion that octocosanol improves the physical resistance through the increase of the hepatic protein synthesis and the conversion of lipids into energy. Octocosanol, included in the nutritional supplementation comprising also aminoacids, vitamins, gamma-orizanol and vegetal porphyrins administered during 8 weeks to 13 athletes (compared with 20 control tests without dietary supplementation) has proved to be capable of reducing the proportion of bodily fat and increasing the circumference of several muscles (biceps, forearm, thigh and calf).

### CARNITINE

Carnitine, whose name is due to the fact that it was isolated for the first time in 1905 from the meat extracts, is an essential factor for transporting the long-chain fatty acids within mitochondrium where such acids are oxidized to ketone bodies and CO₂ for producing energy. It is only present in food of animal origin (above all, meat and milk products) and can be synthesized by the human body in the liver (endogenous carnitine). It cannot then be considered as a vitamin even if Cavter and Coll called it in 1952 vitamin B7, a growth factor of Tenebrio Molitor (meal worm) which dies because of fat progressively accumulating when carnitine is lacking. All of the animals, humans included, are capable of synthesizing carnitine form lysine which constitutes the carbon back-bone, and from methionine which supplies methyl groups; however, they are not said to do it always to a sufficient or optimum extent.

Actually there are several cases in which the amounts synthesized by the organism and supplied by the consumed foods are not sufficient for the normal development of metabolic processes in which the carnitine itself is involved.

This is the case of children brought forth prematurely, in which the carnitine biosynthesis cannot be suitably developed because of the lack of enzymes assigned to produce carnitine, as well as the case of adults keeping to a strictly vegetarian diet (carnitine is not present at all in vegetable foods), and the case of subjects having enhanced requirement of carnitine due to the increased energy demand (sportsmen and athletes). As on one hand the capability of synthesizing carnitine is limited, as mentioned above, and on the other hand the alimentary carnitine is not wholly absorbed but only 54% to 87% of the carnitine included in the normal diets is biologically available, the administration of carnitine may be highly beneficial. In particular, in case of sporting activities it seems that the administration of carnitine can increase the hepatic concentration of ATP and glycogen, as proved in experimental researches with rats.

Double-blind experiments conducted with moderately trained subjects, who were given carnitine in one dose before the beginning of a physical exercise, showed a significant level lowering of the lactic and pyruvic acids in the plasma after the exercise.

Such results are particularly important considering that the lowering of lactate and pyruvate is associated with an increase in the working capability, which means that the administration of carnitine is adapted to increase the working efficiency through a more effective use of the pyruvate and other energy substrates depending on carnitine such as fatty acids and branched aminoacids.

### VITAMIN C

Ascorbic acid has recently gained particular importance for its antioxidizing activity besides its vitaminic activity because of the removal of free radicals. Recent experimental results have proved that ascorbic acid is adapted to protect the lipids and in particular the LDL human plasma fractions from the harmful effects of the oxidation.

It seems that such antioxidizing activity is not only important for preventing atherosclerotic diseases, since a number of oxidation products are involved in its aetiology, but also for regularly carrying out a prolonged physical exercise. Higher levels of malondialdehyde (a parameter for detecting the oxidation state of the organism) have been found in the plasma of subjects after a physical exercise.

Moreover experiments conducted with enzyme preparations and perfused liver of guinea pigs, which were scorbutic because of diets without vitamin C, proved that vitamin C takes part to the biosynthesis of carnitine as co-factor of two enzyme systems of the carnitine biosynthesis. According to the above-described experiments the Applicant has found that the association among octocosanol - carnitine - vitamin C is adapted to supply nutrients capable of improving especially long-range physical performances, thus increasing the working efficiency and the cardiovascular function with a synergic effect capable of enhance the characteristics of the single ingredients.

The Applicant found experimenatlly that the average daily absorption of the three associated active principles by the organism subjected to physical stress should be about 75-130 mg of carnitine, 3-7 mg of octocosanol, and 30-75 mg of vitamin C in order to achieve the desired results. The formulation shown below illustrates the present invention but is not limitative.

Dose unit: 500 mg capsule for oral administration:

| Ingredients | Content of each 500 mg capsule | Content for 100 g |
|---|---|---|
| Maltodextrin | 340 mg | 68 g |
| L-carnitine tartrate | 100 mg | 20 g |
| Octocosanol | 5 mg | 1 g |
| Vitamin C | 50 mg | 10 g |
| Silicon dioxide | 5 mg | 1 g |

### Ingredients of the capsule

Gelatin (USPXX), Dyes.

## Claims

1. Use of the combination of octocosanol, carnitine, and ascorbic acid as dietary energy-giving product.

2. Use of the combination of octocosanol, carnitine, and ascorbic acid according to claim 1, wherein said combination of products is administered to the subject through an alimentary medium in a dose such such as to assure an effective daily absorption.

3. Use of the combination of octocosanol, carnitine, and ascorbic acid according to the preceding claims, wherein the daily absorption of the pure active principles by the organism is about 100 mg carnitine, 5 mg octocosanol, and 50 mg vitamin C.

4. Dietary product useful in nourishing individuals subjected to prolonged physical exercises, comprising the combination of carnitine, octocosanol, and vitamin C in an alimentary medium such as to assure an effective concentration in the organism.

5. Dietary product useful in nourishing individuals subjected to prolonged physical exercises, comprising the combination of carnitine, octocosanol, and vitamin C, wherein the amounts of said active principles in 100 g are 15-25 g carnitine, 0.-5-1.5 g octocosanol, and 5-15 g vitamin C.
